# EUROPÄISCHE PATENTSCHRIFT

(11) **EP 4 159 132 B1**
(45) Veröffentlichungstag und Bekanntmachung des Hinweises auf die Patenterteilung: **05.03.2025**
(21) Anmeldenummer: 22197199.7
(22) Anmeldetag: 22.09.2022
(51) Int. Cl.: A61B 6/02, A61B 6/10, A61B 6/50, A61B 6/00

(54) **MAMMOGRAPHIESYSTEM MIT SCHUTZEINHEIT**
MAMMOGRAPHY SYSTEM WITH PROTECTION UNIT
SYSTÈME DE MAMMOGRAPHIE COMPRENANT UNE UNITÉ DE PROTECTION

(30) Priorität: 30.09.2021 EP 21200287
(43) Veröffentlichungstag der Anmeldung: 05.04.2023
(73) Patentinhaber: Siemens Healthineers AG, 91301 Forchheim (DE)
(72) Erfinder: Limmer, Andreas, 90768 Fürth (DE); Nanke, Ralf, 91077 Neunkirchen am Brand (DE); Radicke, Marcus, 90587 Veitsbronn (DE); Ritter, Juliane, 91096 Möhrendorf (DE); Weidner, Tom, 91056 Erlangen (DE); Eller, Lukas, 91301 Forchheim (DE); Göttler, Stephanie, 92348 Berg (DE); Speitel, Jutta, 91056 Erlangen (DE)
(74) Vertreter: Siemens Healthineers Patent Attorneys

(56) Entgegenhaltungen:
- EP-A1- 2 679 157
- WO-A1-2009/047054
- WO-A1-2020/069031
- DE-A1- 102006 007 833

## Beschreibung

Die Erfindung betrifft ein Mammographiesystem mit Schutzeinheit, welche den Kopf des Untersuchungsobjekts vor Kollisionen mit der Röntgenquelle schützt.

In der röntgenbasierten Brustbildgebung wird die Patientin standardmäßig im Stehen untersucht. Die Brust wird hierbei auf einem Röntgendetektor gelagert; gegenüberliegend zum Detektors befindet sich eine Röntgenröhre die im Falle einer 3D-Aufnahme (Tomosynthese) in Relation zur Detektornormalen ausgelenkt wird. Bei modernen Geräten bewegt die Röntgenröhre sich mit einer Geschwindigkeit von über 2° pro Sekunde. Die Röntgenröhre ist in einem Abstand von etwa 650mm zum Detektor positioniert, was bedeutet, dass für viele Patientinnen die Bewegung der Röhre in Höhe des Kopfes stattfindet. Zwischen bewegter Röhre und Patientenkopf sind aufgrund der notwendigen Positionierung nur wenige cm Abstand. Eine Kollision mit der bewegten Röntgenröhre ist daher theoretisch möglich und sollte möglichst per Hard- und Software des Systems ausgeschlossen werden.

Aus der Druckschrift DE 10 2006 007 833 A1 ist ein Bildgebungsgerät, wie z. B. ein Mammographiegerät, zum Erzeugen radiographischer Bilder und/oder von Bildern eines Objekts, die durch Tomosynthese wiederhergestellt worden sind, bekannt. Diese enthält eine Röntgenquelle, eine Brusthalterung, die zwischen der Röntgenquelle und einem Bildempfänger angeordnet ist, wobei die Röntgenquelle in einem Kopfelement angeordnet ist, das an einem drehbar gelagerten Arm befestigt ist, der von einem ersten Schlitten getragen wird, der an einer Säule gleitet, und wenigstens eine strahlungsdurchlässige Kompressionsplatte, die zwischen der Strahlungsquelle und der Brusthalterung angeordnet und zum Gleiten auf einem zweiten Schlitten angebracht ist, um die Brust einer Patientin zu komprimieren. Das Gerät weist wenigstens einen Schirm zum Schutz des Kopfes oder Halses der Patientin auf, wobei der Schirm in einer festen Stellung, bezogen auf den Kopf oder Hals der Patientin, zwischen der Bahn der Röntgenquelle und dem Kopf oder Hals der Patientin während einer tomographischen Aufnahme, bei der die Röntgenquelle gedreht wird, oder zwischen der Röntgenquelle und dem Kopf oder Hals der Patientin gehalten werden kann, wenn radiographische Bilder aufzunehmen sind, wobei der Schirm zurückgezogen oder entfernt werden kann.

Die WO 2009/044054 A1 beschreibt eine Diagnoseeinheit, insbesondere ein Mammographiegerät oder ein Tomosynthesegerät mit einem zu einem auf einem Objekttisch fixierten Objekt ortsfest befestigbaren Schutzschild, mit welchem eine räumliche Trennung des Patienten und des Röntgenstrahlkopfes erreicht wird.

Es ist Aufgabe der Erfindung, ein Mammographiesystem anzugeben, welches einen komfortablen Schutz der Patientin ermöglicht.

Die Aufgabe wird erfindungsgemäß gelöst durch ein Mammographiesystem nach Anspruch 1.

Die Erfindung kann insbesondere ein Mammographiesystem betreffen, aufweisend:
- eine Standeinheit zur Anordnung des Mammographiesystems auf dem Boden,
- eine L-förmige Quelleneinheit, deren einer Schenkel rotierbar gelagert an einer Vorderseite der Standeinheit mit einem Röntgendetektor verbunden ist und der andere Schenkel im Wesentlichen senkrecht hervorsteht, so dass am vom Standelement bzw. der Standeinheit entfernten Ende der Quelleneinheit eine Röntgenquelle angeordnet ist,
- eine im Wesentlichen U-förmige Schutzeinheit, deren erster Schenkel an der Standeinheit, bevorzugt an einer Rückseite der Standeinheit, unabhängig von der Quelleneinheit rotierbar gelagert befestigt ist, der zweite Schenkel sich in einem ersten Betriebszustand an der Oberseite der Quelleneinheit entlang erstreckt, und der dritte Schenkel am vom Standelement entfernten Ende der Schutzeinheit ein Schutzschild zur Auflage des Patientenkopfes aufweist.

Das Mammographiesystem ist für die Anordnung und Befestigung am Boden, insbesondere eines Untersuchungsraumes, ausgebildet. Die Standeinheit steht auf dem Boden. An der Standeinheit ist eine L-förmige Quelleneinheit angeordnet. Die Quelleneinheit ist drehbar gelagert mit der Standeinheit verbunden, so dass digitale Vollfeldmammographieaufnahmen aus verschiedenen Aufnahmewinkeln, beispielsweise cranio-caudal oder mediolateral-oblique, und Tomosyntheseaufnahmen ermöglicht werden. Die Quelleneinheit ist L-förmig ausgebildet. Ein Schenkel verläuft im Wesentlichen parallel zur Front der Standeinheit und umfasst die drehbar gelagerte Verbindung zur Standeinheit. Ferner ist an der Standeinheit ein Röntgendetektor angeordnet, welcher ebenfalls drehbar gelagert ausgestaltet ist und zwar hinsichtlich der Quelleneinheit als auch der Standeinheit. Damit können die Quelleneinheit und der Röntgendetektor unabhängig voneinander gegenüber der Standeinheit gedreht werden. Die Brust kann auf insbesondere einer Gehäuseoberfläche des Röntgendetektors angeordnet werden. Die Brust kann als Untersuchungsobjekt zwischen der Röntgenquelle und dem Röntgendetektor angeordnet werden. Der Röntgendetektor kann insbesondere während der Röntgenaufnahme stationär sein. Die Röntgenquelle kann für eine digitale Vollfeldmammographieaufnahme gegenüber dem Röntgendetektor angeordnet sein, so dass der Zentralstrahl der Röntgenquelle senkrecht auf die Detektionsfläche des Röntgendetektors trifft. Für eine Tomosyntheseaufnahme kann die Röntgenquelle mittels der Quelleneinheit rotiert werden, insbesondere in einem Winkelbereich von 15 bis 50 Grad. Bei der mittleren Projektion kann der Zentralstrahl senkrecht auf die Detektionsfläche treffen.

Die Schutzeinheit ist U-förmig. Die Schutzeinheit umfasst drei Schenkel. Der erste Schenkel ist an der Standeinheit unabhängig von der Quelleneinheit rotierbar gelagert befestigt.

Der erste Schenkel ist bevorzugt an einer Rückseite der Standeinheit dreh- bzw. rotierbar gelagert befestigt. Der zweite Schenkel erstreckt sich in einem ersten Betriebszustand an der Oberseite der Quelleneinheit entlang. Damit kann der erste und der zweite Schenkel der Formgebung der Rückseite der Standeinheit und der Oberseite der Quelleneinheit folgen. Der dritte Schenkel weist am vom Standelement entfernten Ende der Schutzeinheit ein Schutzschild zur Auflage des Patientenkopfes aufweist. Der dritte Schenkel kann dabei zunächst der Form der Quelleneinheit folgen und dann darüber hinaus fortgesetzt werden, so dass das Schutzschild in einer Kopfhöhe der Patientin bzw. des Patienten ausgebildet ist bzw. ausrichtbar ist. Das Schutzschild kann insbesondere derart ausgestaltet sein, dass diese während der gesamten Aufnahme oder Untersuchung an einem festen Ort verbleibt und der Kopf an dem Schutzschild angelehnt während der gesamten Aufnahme oder Untersuchung verbleiben kann. Der Kopf der Patientin bzw. des Patienten kann damit vorteilhaft geschützt werden, insbesondere vor Kollisionen bei Bewegungen der Quelleneinheit. Vorteilhaft können Ängste vor einer Kollision vorteilhaft reduziert werden, so dass eine angenehmere Untersuchung ermöglicht wird.

Die Schutzeinheit kann insbesondere hinsichtlich der Hardwareschnittstelle, dem Kollisionsschutz und Zusatzfunktionen vorteilhaft verbessert sein. Vorteilhaft kann die Befestigung der Schutzeinheit unabhängig von der Quelleneinheit die Bedienung des Mammographiesystems vereinfachen. Vorteilhaft kann der Kopf verbessert vor Kollisionen bzw. die Angst vor solchen Kollisionen vermindert werden.

In einer Ausführungsform weist das Mammographiesystem eine Standeinheit, eine Quelleneinheit und eine Schutzeinheit auf. Die Standeinheit ist zur Anordnung des Mammographiesystems auf dem Boden ausgelegt. Die L-förmige Quelleneinheit weist einen Schenkel rotierbar gelagert an einer Vorderseite der Standeinheit auf und der andere Schenkel steht im Wesentlichen senkrecht hervor, so dass am von der Standeinheit entfernten Ende der Quelleneinheit eine Röntgenquelle angeordnet ist. An der Vorderseite der Standeinheit ist der Röntgendetektor angeordnet. Der Röntgendetektor kann, insbesondere unabhängig, drehbar gegenüber der Standeinheit bzw. der Quelleneinheit gelagert sein. Die, insbesondere im Wesentlichen U-förmige, Schutzeinheit kann drei Schenkel umfassen. Der erste Schenkel kann von der Standeinheit umfasst sein. Der erste Schenkel kann an einer Rückseite oder einer Vorderseite oder innerhalb der Standeinheit befestigt sein. Der erste Schenkel kann starr oder, insbesondere unabhängig von der Quelleneinheit, rotierbar gelagert befestigt sein. Im Fall, dass der erste Schenkel von der Standeinheit umfasst ist, kann die Schutzeinheit insbesondere starr mit der Standeinheit verbunden sein. Der zweite Schenkel kann sich in einem ersten Betriebszustand an der Oberseite der Quelleneinheit entlang erstrecken. Der dritte Schenkel weist am vom Standelement entfernten Ende der Schutzeinheit ein Schutzschild zur Auflage des Patientenkopfes auf.

Gemäß einem Aspekt der Erfindung ist die Schutzeinheit im Wesentlichen formschlüssig entlang der Rückseite der Standeinheit und entlang der Quelleneinheit in einem ersten Betriebszustand ausgebildet. Die Schutzeinheit folgt zumindest von der rotierbar gelagerten Befestigung an der Standeinheit weiter zur Oberseite der Quelleneinheit und entlang der Oberseite der Quelleneinheit der Form dieser Komponenten. Ein Spalt für ein reibungsfreie Bewegung der Quelleneinheit kann bevorzugt ausgebildet sein.

Gemäß einem Aspekt der Erfindung ist die Schutzeinheit in einem Aufnahmezustand ortsfest. Die Schutzeinheit kann während des Scans (d.h. der Bewegung der Röntgenquelle) ortsfest sein. Die Schutzeinheit kann als Ganzes rotierbar sein. Es kann eine automatische Rotation, je nach eingestelltem View/Aufgabe, oder auch manuell durch die MTRA, durchgeführt werden. Als View kann eine Position, beispielsweise cranio-caudal oder mediolateral-oblique bezeichnet werden. Beispielsweise kann die Schutzeinheit für eine Aufnahme in mediolateral-obliquer Position rotiert werden. Diese Position kann eine Ausrichtung der Schutzeinheit in einem zweiten Betriebszustand beschreiben.

Gemäß einem Aspekt der Erfindung ist das Schutzschild an das Untersuchungsobjekt anpassbar. Die Kontaktfläche zum Patienten kann verschiebbar sein und kann je nach Patientengröße oder je nach View/Aufgabe angepasst werden. Die jeweilige Anpassung kann automatisch, anhand des eingestellten View / der eingestellten Aufgabe, geschehen.

Die Kontaktfläche zum Patienten kann wegklappbar ausgebildet sein. Die Schutzeinheit kann als Ganzes wegklappbar ausgebildet sein. Die Kontaktfläche zum Patienten ist abnehmbar und ggf. austauschbar. Hierbei kann z.B. ein undurchsichtiges Schutzschild gewählt werden, beispielsweise für die Biopsie. Die korrekte Funktion der Schutzeinheit kann visuell kontrolliert werden, z.B. durch Einbau von Lichtleitern, die bei richtigem Einbau ein entsprechendes Leuchten verursachen. In verschiedenen Ausführungsformen kann die Schutzeinheit am Objekttisch, an der Standeinheit oder an einem stationären Zwischenring zwischen der Standeinheit und der Quelleneinheit befestigt werden.

Die Schutzeinheit kann für Transport und Verpackung des Geräts teilbar und abnehmbar ausgebildet sein. Die Schutzeinheit kann eine Vorrichtung, um z.B. an einer Decke (z.B. eines Trucks) befestigt zu werden, umfassen.

Gemäß der Erfindung umfasst die Schutzeinheit Sensoren zum Kollisionsschutz. Die Schutzeinheit kann neben ihrer mechanischen Funktion des Kollisionsschutzes zwischen Patientenkopf und Röntgenquelle einen verbesserten Kollisionsschutz beinhalten. Sensoren, beispielsweise basierend auf Ultraschall, Licht, Schall, oder kapazitive Sensoren können vorgesehen sein, um eine vermeintliche Kollision vor deren Eintritt zu detektieren und das System automatisch zu stoppen. Durch Kraftsensoren kann ein Abbruch der Bewegung der Quelleneinheit bewirkt werden, wenn die seitliche Kraft auf die Schutzeinheit zu groß ist. Hierdurch kann eine mögliche Quetschung verhindert werden, z.B. falls sich ein Finger zwischen Schutzeinheit und Quelleneinheit befindet. Falls die seitliche Kraft auf die Schutzeinheit zu groß wird, kann die Schutzeinheit wegklappen, so dass eine Quetschung verhindert werden kann. Die Zusammenarbeit der Sensoren und des Workflows mit vorhandenen Kameras kann eine frühzeitig Kollisionswarnung ausgebbar machen.

Gemäß einem Aspekt der Erfindung klappt bei einer einen Schwellwert übersteigenden Krafteinwirkung das Schutzschild automatisch weg. Das Schutzschild kann für eine erneute Aufnahme zurück in die Schutzposition gebracht, also zurück in die ursprüngliche Position geklappt, werden.

Gemäß einem Aspekt der Erfindung ist eine Signalverbindung zur Übertragung einer Kollisionsgefahr von der Schutzeinheit an die Steuerung des Röntgensystems ausgebildet. Die Signalverbindung kann kabelgebunden oder kabellos erfolgen.

Die Schutzeinheit kann Zusatzfunktionen aufweisen. Die Schutzeinheit kann ein Moodlight enthalten, um eine angenehme Atmosphäre zu schaffen oder einen Betriebszustand anzuzeigen. Je nach Betriebszustand kann die Schutzeinheit, insbesondere das Schutzschild, in einer anderen Farbe leuchten. Die Beleuchtung kann im Bereich der gesamten Schutzeinheit oder in einem Teilbereich ausgebildet sein. Die Schutzeinheit kann ein Lichtfeld (Arbeitslicht) zur Verfügung stellen. Mittels des Arbeitslichts kann der Untersuchungsraum zwischen der Röntgenquelle und dem Röntgendetektor beleuchtet werden. Insbesondere kann das Arbeitslicht die Oberseite des Röntgendetektors beleuchten, so dass eine Positionierung der Brust vereinfacht wird. Die Schutzeinheit kann nach Koordinatenübermittlung Marker auf die Brust zu projizieren. Vorteilhaft kann die Brust vereinfacht und genauer positioniert werden. Die Schutzeinheit kann in der Lage sein, den Status des Workflows bzw. der Kompression anzuzeigen. Die Schutzeinheit kann dazu ein Display aufweisen. Die Schutzeinheit kann mittels der Beleuchtung den Status des Workflows oder die Kompression anzeigen. Über Präsenzsensoren an der Schutzeinheit kann das Arbeitslicht automatisch eingeschaltet werden. Beispielsweise kann sobald sich ein Patient oder eine Patientin nähert das Arbeitslicht automatisch aktiviert werden. Weiterhin kann der Präsenzsensor die Informationen auf dem Display beeinflussen und zwischen Informationen für den Patienten und für die MTA umschalten. Beispielsweise kann sich je nach Näherungsrichtung das Display auf den Patienten oder die MTA einstellen.

Die Schutzeinheit kann ein Display mit oder ohne TouchOberfläche enthalten oder aus einem solchen Display bestehen. Über die Touchoberfläche kann eine Pulsmessung und/oder eine Temperaturmessung durchgeführt werden. Ein Projektor kann auf die Schutzeinheit Informationen oder auch ein Moodlight projizieren. Über eine Force-Feedback Funktion können dem Patienten oder der MTA Informationen über Vibrationen mitgeteilt werden. Die Schutzeinheit kann über eine automatische oder semi-automatische Selbstreinigung (automatische Desinfektion) verfügen. Die Schutzeinheit kann einen automatischer Rollenhalter beinhalten, so dass bei einem Patientenwechsel jeweils die Kontaktfläche ausgetauscht wird (Kontaktfläche besteht aus Papier, Tuch oder Folie und wird über ein festes Material ohne Patientenkontakt stabilisiert). Über einen Sensor wird die Lage / Ausrichtung der Schutzeinheit kontrolliert und eventuell eine Warnung ausgegeben (falls der eingestellte Workflow nicht zur Position des Faceshields passt).

Gemäß einem Aspekt der Erfindung umfasst die Schutzeinheit eine Beleuchtungseinheit. Die Beleuchtungseinheit kann ein Moodlight, also eine Stimmungsbeleuchtung, eine Beleuchtung entsprechend des Betriebszustands, einer Kompression oder eines Workflowstatus oder eine Arbeitsbeleuchtung umfassen.

Gemäß einem Aspekt der Erfindung umfasst die Schutzeinheit eine Anzeigeeinheit und/oder Eingabeeinheit. Die Anzeigeeinheit und/oder die Eingabeeinheit können insbesondere am Schutzschild oder oberhalb davon im Bereich der Röntgenquelle an der Quelleneinheit ausgebildet sein.

Gemäß einem Aspekt der Erfindung ist ein Präsenzsensor zur Unterscheidung von Untersuchungsobjekt und Benutzer vorgesehen. Der Präsenzsensor kann insbesondere am Schutzschild oder oberhalb davon im Bereich der Röntgenquelle an der Quelleneinheit ausgebildet sein.

Gemäß einem Aspekt der Erfindung umfasst die Schutzeinheit eine Reinigungseinheit. Die Reinigungseinheit kann insbesondere zur Reinigung des Schutzschilds ausgebildet sein. Die Reinigungseinheit kann eine Reinigung mittels UV-Licht oder Desinfektionsmittel bereitstellen.

Gemäß einem Aspekt der Erfindung ist ein Lagesensor der Schutzeinheit mit der Steuerung des Röntgensystems verbunden. Es kann damit ein Signal bezüglich des Betriebszustands, der Kompression oder des Workflowstatus übertragen werden. Beispielsweise kann mit diesem Signal die Beleuchtung gesteuert werden.

Gemäß einem Aspekt der Erfindung weist das Schutzschild eine konvexe Krümmung hin zum Untersuchungsobjekt auf. Damit kann das Schutzschild im Wesentlichen an eine Kopfform angepasst sein. Vorteilhaft kann der Komfort für die Patientin bzw. den Patienten erhöht werden. Vorteilhaft kann ein seitliches Abrutschen des Kopfes vom Schutzschild weg vermieden werden.

Gemäß einem Aspekt der Erfindung ist wobei die Form des Schutzschildes an die Kopfform angepasst.

Gemäß einem Aspekt der Erfindung umfasst das Schutzschild einen Temperatursensor oder/und einen Pulssensor. Vorteilhaft kann ein Zustand des Patienten bzw. der Patientin ermittelt werden. Der Zustand kann sich auf ein Stresslevel beziehen, insbesondere in Bezug auf den Pulssensor. Die Messwerte des Temperatursensor oder/und des Pulssensors können der MTRA mitgeteilt werden, beispielsweise über eine Anzeigeeinheit oder auch akustisch oder optisch, insbesondere über die Beleuchtungseinheit. Die Messwerte des Temperatursensor oder/und des Pulssensors können gemeinsam mit der Aufnahme gespeichert werden.

Insbesondere das Schutzschild kann ein neuartige Form aufweisen. Die Krümmung des Schutzschilds zur Patientin hin, kann so ausgebildet sein, dass die Frau den Kopf wie in einer Schale lagern kann. Die Frau kann ihren Kopf nur schwer am Schutzschild vorbei anlehnen und die Haare sollten auch nicht in den Strahlengang oder in die Röhrenbewegung kommen. Die Krümmung und Breite des Schutzschild kann so gestaltet sein, dass die Frau den Kopf bequem anlehnen kann, ohne sich eingeengt zu fühlen. Das Schutzschild kann entweder rausgenommen oder weggeschoben werden, z.B. für Untersuchungen, bei denen es nicht gebraucht wird. Durch die Krümmung nach außen, kann die Frau ihren Kopf gesichert und bequem anlehnen. Der Vorteil dieses Schutzschilds ist es, dass es stationär ist und damit bei allen Untersuchungen verwendet werden kann, auch für schräge Aufnahmen, es werden keine weiteren Schutzschilder benötigt.

Nachfolgend werden Ausführungsbeispiele der Erfindung anhand von Zeichnungen näher erläutert. Hierbei zeigt:
FIG 1 schematisch eine Darstellung eines erfindungsgemä-ßen Röntgensystems in einer ersten Ausführungsform;
FIG 2 schematisch eine Darstellung eines erfindungsgemä-ßen Röntgensystems in einer zweiten Ausführungsform;
FIG 3 schematisch eine Darstellung eines erfindungsgemä-ßen Röntgensystems in einer dritten Ausführungsform;
FIG 4 schematisch eine Darstellung eines erfindungsgemä-ßen Röntgensystems in einer vierten Ausführungsform;
FIG 5 schematisch eine Darstellung eines erfindungsgemä-ßen Röntgensystems in einer fünften Ausführungsform;
FIG 6 schematisch eine Darstellung eines erfindungsgemä-ßen Röntgensystems in einer sechsten Ausführungsform, und
FIG 7 schematisch eine Darstellung eines erfindungsgemä-ßen Röntgensystems in einer siebten Ausführungsform.

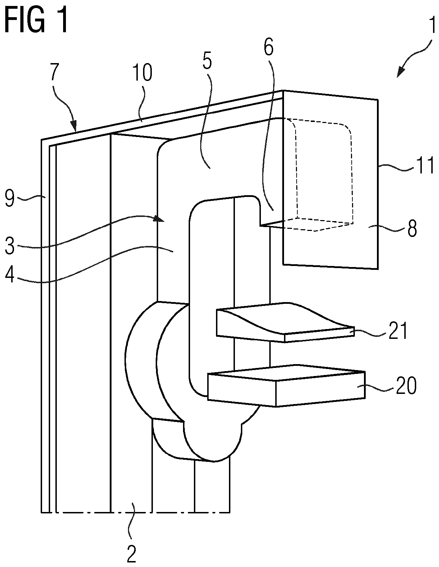

Die Fig. 1 zeigt eine beispielhafte Ausführung eines erfindungsgemäßen Röntgensystems 1 in einer ersten Ausführungsform. Das Mammographiesystem 1 weist eine Standeinheit 2 zur Anordnung des Mammographiesystems 1 auf dem Boden auf. Das Mammographiesystem 1 weist ferner eine L-förmige Quelleneinheit 3 auf, deren einer Schenkel 4 rotierbar gelagert an einer Vorderseite der Standeinheit 2 mit dem Röntgendetektor 20 verbunden ist und der andere Schenkel 5 im Wesentlichen senkrecht hervorsteht, so dass am vom Standelement bzw. der Standeinheit entfernten Ende der Quelleneinheit 3 eine Röntgenquelle 6 angeordnet ist. Das Mammographiesystem 1 weist ferner eine im Wesentlichen U-förmige Schutzeinheit 7 auf, deren erster Schenkel 9 an einer Rückseite der Standeinheit 2 unabhängig von der Quelleneinheit 3 rotierbar gelagert befestigt ist, der zweite Schenkel 10 sich in einem ersten Betriebszustand an der Oberseite der Quelleneinheit 3 entlang erstreckt, und der dritte Schenkel 11 am vom Standelement bzw. der Standeinheit 2 entfernten Ende der Schutzeinheit 7 ein Schutzschild 8 zur Auflage des Patientenkopfes aufweist.

Die Schutzeinheit 7 ist im Wesentlichen formschlüssig entlang der Rückseite der Standeinheit 2 und entlang der Quelleneinheit 3 in einem ersten Betriebszustand ausgebildet. Die Schutzeinheit 7 in einem Aufnahmezustand ortsfest.

Das Mammographiesystem 1 ist für die Anordnung und Befestigung am Boden, insbesondere eines Untersuchungsraumes, ausgebildet. Die Standeinheit 2 steht auf dem Boden. An der Standeinheit 2 ist eine L-förmige Quelleneinheit 3 angeordnet. Die Quelleneinheit 3 ist drehbar gelagert mit der Standeinheit 2 verbunden, so dass digitale Vollfeldmammographieaufnahmen aus verschiedenen Aufnahmewinkeln, beispielsweise cranio-caudal oder mediolateral-oblique, und Tomosyntheseaufnahmen ermöglicht werden. Die Quelleneinheit 3 ist L-förmig ausgebildet. Ein Schenkel 4 verläuft im Wesentlichen parallel zur Front der Standeinheit 2 und umfasst die drehbar gelagerte Verbindung zur Standeinheit 2. Ferner ist an der Standeinheit 2 ein Röntgendetektor 20 angeordnet, welcher ebenfalls drehbar gelagert ausgestaltet ist und zwar hinsichtlich der Quelleneinheit 3 als auch der Standeinheit 2. Damit können die Quelleneinheit 3 und der Röntgendetektor 20 unabhängig voneinander gegenüber der Standeinheit 2 gedreht werden. Die Brust kann auf insbesondere einer Gehäuseoberfläche des Röntgendetektors 20 angeordnet werden. Die Brust kann als Untersuchungsobjekt zwischen der Röntgenquelle 6 und dem Röntgendetektor 20 angeordnet werden. Der Röntgendetektor 20 kann insbesondere während der Röntgenaufnahme stationär sein. Die Röntgenquelle 6 kann für eine digitale Vollfeldmammographieaufnahme gegenüber dem Röntgendetektor 20 angeordnet sein, so dass der Zentralstrahl der Röntgenquelle 6 senkrecht auf die Detektionsfläche des Röntgendetektors 20 trifft. Für eine Tomosyntheseaufnahme kann die Röntgenquelle 6 mittels der Quelleneinheit 3 rotiert werden, insbesondere in einem Winkelbereich von 15 bis 50 Grad. Bei der mittleren Projektion kann der Zentralstrahl senkrecht auf die Detektionsfläche treffen.

Die Schutzeinheit 7 ist U-förmig. Die Schutzeinheit 7 umfasst drei Schenkel 9, 10, 11. Der erste Schenkel 9 ist an der Standeinheit 2 unabhängig von der Quelleneinheit 3 rotierbar gelagert befestigt. Der erste Schenkel 9 ist bevorzugt an einer Rückseite der Standeinheit 2 dreh- bzw. rotierbar gelagert befestigt. Der zweite Schenkel 10 erstreckt sich in einem ersten Betriebszustand an der Oberseite der Quelleneinheit 3 entlang. Damit kann der erste Schenkel 9 und der zweite Schenkel 10 der Formgebung der Rückseite der Standeinheit 2 und der Oberseite der Quelleneinheit 3 folgen. Der dritte Schenkel 11 weist am vom Standelement bzw. der Standeinheit 2 entfernten Ende der Schutzeinheit 7 ein Schutzschild 8 zur Auflage des Patientenkopfes auf. Der dritte Schenkel 11 kann dabei zunächst der Form der Quelleneinheit 3 folgen und dann darüber hinaus fortgesetzt werden, so dass das Schutzschild 8 in einer Kopfhöhe der Patientin bzw. des Patienten ausgebildet ist bzw. ausrichtbar ist. Das Schutzschild 8 kann insbesondere derart ausgestaltet sein, dass diese während der gesamten Aufnahme oder Untersuchung an einem festen Ort verbleibt und der Kopf an dem Schutzschild 8 angelehnt während der gesamten Aufnahme oder Untersuchung verbleiben kann.

Die Schutzeinheit 7 ist im Wesentlichen formschlüssig entlang der Rückseite der Standeinheit 2 und entlang der Quelleneinheit 3 in einem ersten Betriebszustand ausgebildet. Die Schutzeinheit 7 folgt zumindest von der rotierbar gelagerten Befestigung an der Standeinheit 2 weiter zur Oberseite der Quelleneinheit 3 und entlang der Oberseite der Quelleneinheit 3 der Form dieser Komponenten. Ein Spalt für ein reibungsfreie Bewegung der Quelleneinheit 3 kann bevorzugt ausgebildet sein.

Die Schutzeinheit 7 ist in einem Aufnahmezustand ortsfest. Die Schutzeinheit 7 kann während des Scans (d.h. der Bewegung der Röntgenquelle) ortsfest sein. Die Schutzeinheit 7 kann als Ganzes rotierbar sein. Es kann eine automatische Rotation, je nach eingestelltem View/Aufgabe, oder auch manuell durch die MTRA, durchgeführt werden. Als View kann eine Position, beispielsweise cranio-caudal oder mediolateral-oblique bezeichnet werden. Beispielsweise kann die Schutzeinheit 7 für eine Aufnahme in mediolateral-obliquer Position rotiert werden. Diese Position kann eine Ausrichtung der Schutzeinheit 7 in einem zweiten Betriebszustand beschreiben.

Das Schutzschild 8 an das Untersuchungsobjekt anpassbar. Die Kontaktfläche zum Patienten kann verschiebbar sein und kann je nach Patientengröße oder je nach View/Aufgabe angepasst werden. Die jeweilige Anpassung kann automatisch, anhand des eingestellten View / der eingestellten Aufgabe, geschehen.

Die Kontaktfläche zum Patienten kann wegklappbar ausgebildet sein. Die Schutzeinheit 7 kann als Ganzes wegklappbar ausgebildet sein. Die Kontaktfläche zum Patienten ist abnehmbar und ggf. austauschbar. Hierbei kann z.B. ein undurchsichtiges Schutzschild 8 gewählt werden, beispielsweise für die Biopsie.

Die korrekte Funktion der Schutzeinheit 7 kann visuell kontrolliert werden, z.B. durch Einbau von Lichtleitern, die bei richtigem Einbau ein entsprechendes Leuchten verursachen. In verschiedenen Ausführungsformen kann die Schutzeinheit 7 am Objekttisch, an der Standeinheit 2 oder an einem stationären Zwischenring zwischen der Standeinheit 2 und der Quelleneinheit 3 befestigt werden.

Die Schutzeinheit 7 kann für Transport und Verpackung des Geräts teilbar und abnehmbar ausgebildet sein. Die Schutzeinheit 7 kann eine Vorrichtung, um z.B. an einer Decke (z.B. eines Trucks) befestigt zu werden, umfassen.

Die Schutzeinheit 7 umfasst Sensoren zum Kollisionsschutz. Die Schutzeinheit 7 kann neben ihrer mechanischen Funktion des Kollisionsschutzes zwischen Patientenkopf und Röntgenquelle 6 einen verbesserten Kollisionsschutz beinhalten. Sensoren, beispielsweise basierend auf Ultraschall, Licht, Schall, oder kapazitive Sensoren können vorgesehen sein, um eine vermeintliche Kollision vor deren Eintritt zu detektieren und das System automatisch zu stoppen. Durch Kraftsensoren kann ein Abbruch der Bewegung der Quelleneinheit 3 bewirkt werden, wenn die seitliche Kraft auf die Schutzeinheit 7 zu groß ist. Hierdurch kann eine mögliche Quetschung verhindert werden, z.B. falls sich ein Finger zwischen Schutzeinheit 7 und Quelleneinheit 3 befindet. Falls die seitliche Kraft auf die Schutzeinheit 7 zu groß wird, kann die Schutzeinheit 7 wegklappen, so dass eine Quetschung verhindert werden kann. Die Zusammenarbeit der Sensoren und des Workflows mit vorhandenen Kameras kann eine frühzeitig Kollisionswarnung ausgebbar machen.

Bei einer einen Schwellwert übersteigenden Krafteinwirkung klappt das Schutzschild 8 automatisch weg. Das Schutzschild 8 kann für eine erneute Aufnahme zurück in die Schutzposition gebracht, also zurück in die ursprüngliche Position geklappt, werden.

Eine Signalverbindung zur Übertragung einer Kollisionsgefahr von der Schutzeinheit 7 an die Steuerung des Röntgensystems ist ausgebildet. Die Signalverbindung kann kabelgebunden oder kabellos erfolgen.

Die Schutzeinheit 7 kann Zusatzfunktionen aufweisen. Die Schutzeinheit 7 kann ein Moodlight enthalten, um eine angenehme Atmosphäre zu schaffen oder einen Betriebszustand anzuzeigen. Je nach Betriebszustand kann die Schutzeinheit 7, insbesondere das Schutzschild 8, in einer anderen Farbe leuchten. Die Beleuchtung kann im Bereich der gesamten Schutzeinheit 7 oder in einem Teilbereich ausgebildet sein. Die Schutzeinheit 7 kann ein Lichtfeld (Arbeitslicht) zur Verfügung stellen. Mittels des Arbeitslichts kann der Untersuchungsraum zwischen der Röntgenquelle 6 und dem Röntgendetektor 20 beleuchtet werden. Insbesondere kann das Arbeitslicht die Oberseite des Röntgendetektors 20 beleuchten, so dass eine Positionierung der Brust vereinfacht wird. Die Schutzeinheit 7 kann nach Koordinatenübermittlung Marker auf die Brust zu projizieren. Vorteilhaft kann die Brust vereinfacht und genauer positioniert werden. Die Schutzeinheit 7 kann in der Lage sein, den Status des Workflows bzw. der Kompression anzuzeigen. Die Schutzeinheit 7 kann dazu ein Display aufweisen. Die Schutzeinheit 7 kann mittels der Beleuchtung den Status des Workflows oder die Kompression anzeigen. Über Präsenzsensoren an der Schutzeinheit 7 kann das Arbeitslicht automatisch eingeschaltet werden. Beispielsweise kann sobald sich ein Patient oder eine Patientin nähert das Arbeitslicht automatisch aktiviert werden. Weiterhin kann der Präsenzsensor die Informationen auf dem Display beeinflussen und zwischen Informationen für den Patienten und für die MTA umschalten. Beispielsweise kann sich je nach Näherungsrichtung das Display auf den Patienten oder die MTA einstellen.

Die Schutzeinheit 7 kann ein Display mit oder ohne TouchOberfläche enthalten oder aus einem solchen Display bestehen. Über die Touchoberfläche kann eine Pulsmessung und/oder eine Temperaturmessung durchgeführt werden. Ein Projektor kann auf die Schutzeinheit 7 Informationen oder auch ein Moodlight projizieren. Über eine Force-Feedback Funktion können dem Patienten oder der MTA Informationen über Vibrationen mitgeteilt werden. Die Schutzeinheit 7 kann über eine automatische oder semi-automatische Selbstreinigung (automatische Desinfektion) verfügen. Die Schutzeinheit 7 kann einen automatischer Rollenhalter beinhalten, so dass bei einem Patientenwechsel jeweils die Kontaktfläche ausgetauscht wird (Kontaktfläche besteht aus Papier, Tuch oder Folie und wird über ein festes Material ohne Patientenkontakt stabilisiert). Über einen Sensor wird die Lage / Ausrichtung der Schutzeinheit 7 kontrolliert und eventuell eine Warnung ausgegeben (falls der eingestellte Workflow nicht zur Position des Faceshields passt).

Die Schutzeinheit 7 umfasst eine Beleuchtungseinheit. Die Beleuchtungseinheit kann ein Moodlight, also eine Stimmungsbeleuchtung, eine Beleuchtung entsprechend des Betriebszustands, einer Kompression oder eines Workflowstatus oder eine Arbeitsbeleuchtung umfassen.

Die Schutzeinheit 7 umfasst eine Anzeigeeinheit und/oder Eingabeeinheit. Die Anzeigeeinheit und/oder die Eingabeeinheit können insbesondere am Schutzschild 8 oder oberhalb davon im Bereich der Röntgenquelle 6 an der Quelleneinheit 3 ausgebildet sein.

Ein Präsenzsensor ist zur Unterscheidung von Untersuchungsobjekt und Benutzer vorgesehen. Der Präsenzsensor kann insbesondere am Schutzschild 8 oder oberhalb davon im Bereich der Röntgenquelle 6 an der Quelleneinheit 3 ausgebildet sein.

Die Schutzeinheit 7 umfasst eine Reinigungseinheit. Die Reinigungseinheit kann insbesondere zur Reinigung des Schutzschilds 8 ausgebildet sein. Die Reinigungseinheit kann eine Reinigung mittels UV-Licht oder Desinfektionsmittel bereitstellen.

Ein Lagesensor der Schutzeinheit 7 ist mit der Steuerung des Röntgensystems bzw. des Mammographiesystems 1 verbunden. Es kann damit ein Signal bezüglich des Betriebszustands, der Kompression oder des Workflowstatus übertragen werden. Beispielsweise kann mit diesem Signal die Beleuchtung gesteuert werden.

Das Schutzschild 8 weist eine konvexe Krümmung hin zum Untersuchungsobjekt auf. Damit kann das Schutzschild 8 im Wesentlichen an eine Kopfform angepasst sein. Die Form des Schutzschildes 8 ist an die Kopfform angepasst.

Das Schutzschild 8 umfasst einen Temperatursensor oder/und einen Pulssensor. Der Zustand kann sich auf ein Stresslevel beziehen, insbesondere in Bezug auf den Pulssensor. Die Messwerte des Temperatursensor oder/und des Pulssensors können der MTRA mitgeteilt werden, beispielsweise über eine Anzeigeeinheit oder auch akustisch oder optisch, insbesondere über die Beleuchtungseinheit. Die Messwerte des Temperatursensor oder/und des Pulssensors können gemeinsam mit der Aufnahme gespeichert werden.

Insbesondere das Schutzschild 8 kann ein neuartige Form aufweisen. Die Krümmung des Schutzschilds 8 zur Patientin hin, kann so ausgebildet sein, dass die Frau den Kopf wie in einer Schale lagern kann. Die Frau kann ihren Kopf nur schwer am Schutzschild 8 vorbei anlehnen und die Haare sollten auch nicht in den Strahlengang oder in die Röhrenbewegung kommen. Die Krümmung und Breite des Schutzschild 8 kann so gestaltet sein, dass die Frau den Kopf bequem anlehnen kann, ohne sich eingeengt zu fühlen. Das Schutzschild 8 kann entweder rausgenommen oder weggeschoben werden, z.B. für Untersuchungen, bei denen es nicht gebraucht wird. Durch die Krümmung nach außen, kann die Frau ihren Kopf gesichert und bequem anlehnen. Der Vorteil dieses Schutzschilds 8 ist es, dass es stationär ist und damit bei allen Untersuchungen verwendet werden kann, auch für schräge Aufnahmen, es werden keine weiteren Schutzschilder 8 benötigt.

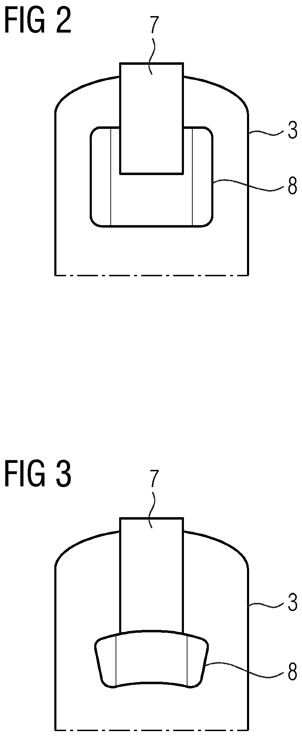

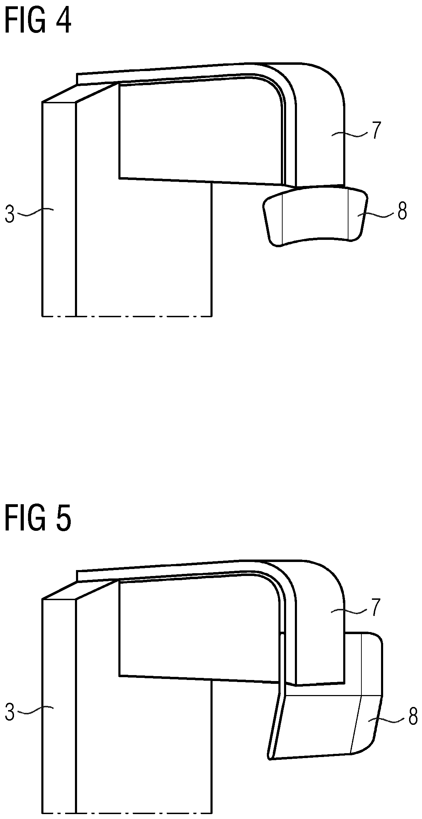

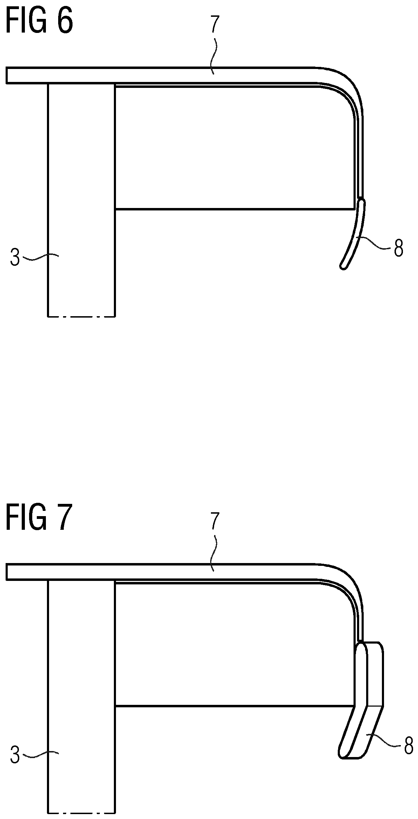

Die Fig. 2 bis 7 zeigen eine beispielhafte Ausführungen eines erfindungsgemäßen Röntgensystems bzw. Mammographiesystems in einer zweiten bis siebten Ausführungsform. Das Schutzschild 8 weist eine konvexe Krümmung hin zum Untersuchungsobjekt auf. Die Form des Schutzschildes 8 ist an die Kopfform angepasst. Die Figuren 2, 5 und 7 zeigen unterschiedliche Perspektiven einer beispielhaften ersten Ausgestaltung des Schutzschildes 8. Die Figuren 3, 4 und 6 zeigen unterschiedliche Perspektiven einer beispielhaften zweiten Ausgestaltung des Schutzschildes 8.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Mammographiesystem (1) aufweisend
**a.** eine Standeinheit (2) zur Anordnung des Mammographiesystems auf dem Boden,
**b.** eine L-förmige Quelleneinheit (3), deren einer Schenkel (4) rotierbar gelagert an einer Vorderseite der Standeinheit mit einem Röntgendetektor (20) verbunden ist und der andere Schenkel (5) im Wesentlichen senkrecht hervorsteht, so dass am von der Standeinheit entfernten Ende der Quelleneinheit eine Röntgenquelle (6) angeordnet ist,
**c.** eine im Wesentlichen U-förmige Schutzeinheit (7), deren erster Schenkel (9) an, insbesondere einer Rückseite, der Standeinheit, insbesondere unabhängig von der Quelleneinheit rotierbar gelagert, befestigt ist, der zweite Schenkel (10) sich in einem ersten Betriebszustand an der Oberseite der Quelleneinheit entlang erstreckt, und der dritte Schenkel (11) am vom Standelement entfernten Ende der Schutzeinheit ein Schutzschild (8) zur Auflage des Patientenkopfes aufweist,
**dadurch gekennzeichnet, dass** die Schutzeinheit Sensoren zum Kollisionsschutz umfasst.

2. Mammographiesystem nach Anspruch 1, wobei die Schutzeinheit im Wesentlichen formschlüssig entlang der Rückseite der Standeinheit und entlang der Quelleneinheit in einem ersten Betriebszustand ausgebildet ist.

3. Mammographiesystem nach einem der vorangehenden Ansprüche, wobei die Schutzeinheit in einem Aufnahmezustand ortsfest ist.

4. Mammographiesystem nach einem der vorangehenden Ansprüche, wobei das Schutzschild an das Untersuchungsobjekt anpassbar ist.

5. Mammographiesystem nach einem der vorangehenden Ansprüche, wobei bei einer einen Schwellwert übersteigenden Krafteinwirkung das Schutzschild automatisch wegklappt.

6. Mammographiesystem nach einem der vorangehenden Ansprüche, wobei eine Signalverbindung zur Übertragung einer Kollisionsgefahr von der Schutzeinheit an die Steuerung des Mammographiesystems ausgebildet ist.

7. Mammographiesystem nach einem der vorangehenden Ansprüche, wobei die Schutzeinheit eine Beleuchtungseinheit umfasst.

8. Mammographiesystem nach einem der vorangehenden Ansprüche, wobei die Schutzeinheit eine Anzeigeeinheit und/oder Eingabeeinheit umfasst.

9. Mammographiesystem nach Anspruch 8, wobei ein Präsenzsensor zur Unterscheidung von Untersuchungsobjekt und Benutzer vorgesehen ist.

10. Mammographiesystem nach einem der vorangehenden Ansprüche, wobei die Schutzeinheit eine Reinigungseinheit umfasst.

11. Mammographiesystem nach einem der vorangehenden Ansprüche, wobei ein Lagesensor der Schutzeinheit mit der Steuerung des Mammographiesystems verbunden ist.

12. Mammographiesystem nach einem der vorangehenden Ansprüche, wobei das Schutzschild eine konvexe Krümmung hin zum Untersuchungsobjekt aufweist.

13. Mammographiesystem nach Anspruch 12, wobei die Form des Schutzschildes an die Kopfform angepasst ist.

14. Mammographiesystem nach einem der vorangehenden Ansprüche, wobei das Schutzschild einen Temperatursensor oder/und einen Pulssensor umfasst.

## Claims

1. Mammography system (1) having
**a.** a stand unit (2) for arrangement of the mammography system on the floor;
**b.** an L-shaped source unit (3), one arm (4) of which is connected to an X-ray detector (20) in a rotatably mounted manner on a front face of the stand unit, and the other arm (5) protrudes substantially perpendicularly, with the result that an X-ray source (6) is arranged at the end of the source unit remote from the stand unit;
c. a substantially U-shaped protective unit (7), the first arm (9) of which is attached to the stand unit, in particular to a rear face thereof, in a rotatably mounted manner in particular such that it can rotate independently of the source unit, the second arm (10) extends along the top face of the source unit in a first operating state, and the third arm (11) has, at the end of the protective unit remote from the stand element, a protective shield (8) for supporting the patient's head,
**characterised in that** the protective unit comprises sensors for collision protection.

2. Mammography system according to claim 1, wherein the protective unit is designed to be substantially form-fitting along the rear face of the stand unit and along the source unit in a first operating state.

3. Mammography system according to one of the preceding claims, wherein the protective unit is stationary in an acquisition state.

4. Mammography system according to one of the preceding claims, wherein the protective shield is adjustable to suit the object under examination.

5. Mammography system according to one of the preceding claims, wherein the protective shield pivots away automatically in the event that an applied force exceeds a threshold value.

6. Mammography system according to one of the preceding claims, wherein a signal connection is designed to transmit a collision risk from the protective unit to the controller of the mammography system.

7. Mammography system according to one of the preceding claims, wherein the protective unit comprises a lighting unit.

8. Mammography system according to one of the preceding claims, wherein the protective unit comprises a display unit and/or input unit.

9. Mammography system according to claim 8, wherein a presence sensor for distinguishing between object under examination and user is provided.

10. Mammography system according to one of the preceding claims, wherein the protective unit comprises a cleaning unit.

11. Mammography system according to one of the preceding claims, wherein a position sensor of the protective unit is connected to the controller of the mammography system.

12. Mammography system according to one of the preceding claims, wherein the protective shield has a convex curvature towards the object under examination.

13. Mammography system according to claim 12, wherein the shape of the protective shield conforms to the shape of the head.

14. Mammography system according to one of the preceding claims, wherein the protective shield comprises a temperature sensor and/or a heart rate sensor.

## Revendications

1. Système (1) de mammographie comportant
a. une unité (2) de mise en place pour le montage du système de mammographie sur le sol,
b. une unité (3) de source en forme de L, dont l'une des branches (4), montée tournante d'un côté avant de l'unité de mise en place, est assemblée à un détecteur (20) de rayons X et dont l'autre branche (5) fait saillie sensiblement perpendiculairement, de manière à monter une source (6) de rayons X à l'extrémité de l'unité de source loin de l'unité de mise en place,
c. une unité (7) de protection sensiblement en forme de U, dont la première branche (9) est fixée à l'unité de mise en place, en particulier à un côté arrière, en étant montée tournante, en particulier indépendamment de l'unité de source, dont la deuxième branche (10) s'étend dans un premier état de fonctionnement le long du côté supérieur de l'unité de source et dont la troisième branche (11) a, pour le support de la tête du patient, un écran (8) de protection à l'extrémité de l'unité de protection loin de l'élément de mise en place,
**caractérisé en ce que**
l'unité de protection comprend des capteurs de protection vis-à-vis d'une collision.

2. Système de mammographie suivant la revendication 1, dans lequel l'unité de protection est constituée, dans un premier état de fonctionnement, sensiblement à complémentarité de forme le long du côté arrière de l'unité de mise en place et le long de l'unité de source.

3. Système de mammographie suivant l'une des revendications précédentes, dans lequel l'unité de protection est fixe en emplacement dans un état de réception.

4. Système de mammographie suivant l'une des revendications précédentes, dans lequel l'écran de protection peut être adapté à l'objet à examiner.

5. Système de mammographie suivant l'une des revendications précédentes, dans lequel, lorsqu'une force déplaçant une valeur de seuil est appliquée, l'écran de protection s'écarte automatiquement.

6. Système de mammographie suivant l'une des revendications précédentes, dans lequel une liaison de signal est constituée pour la transmission d'un danger de collision de l'unité de protection à la commande du système de mammographie.

7. Système de mammographie suivant l'une des revendications précédentes, dans lequel l'unité de protection comprend une unité d'éclairage.

8. Système de mammographie suivant l'une des revendications précédentes, dans lequel l'unité de protection comprend une unité d'affichage et/ou une unité d'entrée.

9. Système de mammographie suivant la revendication 8, dans lequel un capteur de présence est prévu pour la distinction entre un objet à examiner et un utilisateur.

10. Système de mammographie suivant l'une des revendications précédentes, dans lequel l'unité de protection comprend une unité de nettoyage.

11. Système de mammographie suivant l'une des revendications précédentes, dans lequel un capteur de position de l'unité de protection est relié à la commande du système de mammographie.

12. Système de mammographie suivant l'une des revendications précédentes, dans lequel l'écran de protection tourne sa convexité vers l'objet à examiner.

13. Système de mammographie suivant la revendication 12, dans lequel la forme de l'écran de protection est adaptée à la forme de la tête.

14. Système de mammographie suivant l'une des revendications précédentes, dans lequel l'écran de protection comprend une sonde de température ou/et un capteur de pouls.
